# EUROPEAN PATENT APPLICATION

(11) **EP 2 258 696 A1**
(43) Date of publication of application: **08.12.2010**
(21) Application number: 09425187.3
(22) Date of filing: 14.05.2009
(51) Int. Cl.: C07D 317/54, C11D 3/50

(54) **Process for preparing enriched enantiomers of 3-(Benzo[1,3]dioxol-5-yl)-2-methylpropanal**

(71) Applicant: ENDURA S.p.A., 40121 Bologna (IT)
(72) Inventor: Borzatta, Valerio, 40127 Bologna (IT); Rosini, Goffredo, 40135 Bologna (IT); Righi, Paolo, 40100 Bologna (IT)
(74) Representative: Cattaneo, Elisabetta

(57) **Abstract**

A process is described for preparing enriched enantiomers of the compound 3-(benzo[1,3]dioxol-5-yl)-2-methylpropanal of formula (I) comprising the following steps: i) reacting the racemic compound 3-(benzo[1,3]dioxol-5-yl)-2-methylpropanol (2) with phthalic anhydride to give the racemic acid (3); ii) resolving the racemic acid (3) with optically active 1-aryl-1-ethanamine (4) in which Ar is an aromatic or heteroaromatic group having from 6 to 12 members optionally substituted with one or more substituents chosen from the group consisting of (C₁-C₃) alkyl, (C₁-C₃) alkoxy and halogen; iii) recovering the (S)-(2) and (R)-(2) alcohols from the resolved acids (3) of step ii) by alkaline hydrolysis; iv) converting the (S)-2 and (R)-2 alcohols into the respective (S) and (R) enantiomers of compound (I).

## Description

### FIELD OF THE INVENTION

The present invention relates to a process for preparing enriched enantiomers of the compound 3-(benzo[1,3]dioxol-5-yl)-2-methylpropanal (I) of formula:

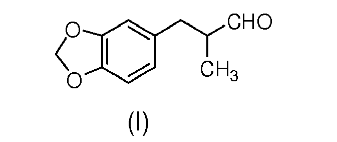

### STATE OF THE ART

Among aldehyde fragrances, the compound 3-(benzo[1,3]dioxol-5-yl)-2-methylpropanal, also known by different trade names such as Tropional® or Helional®, is a commercially important fragrance used in many perfumes. It exhibits a green-floral odour profile (cyclamen) with a fresh, marine, ozone and fresh-cut grass top note (K. Bauer, D. Garbe, H. Surburg Common Fragrance and Flavor Materials; Wiley-VCH, 1997; P. Kraft et al. Angew. Chem. Int. Ed. 2000, 39, 2980; E. Brenna, C. Fuganti, S. Serra Tetrahedron: Asymmetry 2003, 14, 1-42; M. Shirai et al. (Ube), WO 054997, 2004).

Many processes are known for preparing the compound 3-(benzo[1,3]dioxol-5-yl)-2-methylpropanal (1) as a raceme [(a) M. Shirai, Y. Yoshida (UBE), JP 2004269376; (b) W. Chen et al. (Hangzhou Gelin Perfume Chemistry Co.) CN 1365962, 2002 (C.A. 140, 16717); (c) T. Kawanobe et al. (Hasegawa Koryo Co.), JP 10120674, 1998 (C.A. 129,16115); (d) K. Burns et al. (Hoechst), EP 0477747,1992 (C.A. 117, 14232); (e) A. C. da Silva et al., J. Mol. Catal. A: Chem. 2002,179, 133], the annual production of which is estimated at more than 300 tonnes.

The synthesis of both enantiomers of Helional was carried out in 2004 (D. Enders, M. Backes, Tetrahedron: Asymmetry, 2004,15,1813) by asymmetric alkylation using the SAMP/RAMP-hydrazone method developed by said Enders. The alkylated hydrazones are oxidatively cleaved with magnesium monoperoxyphthalate (MMPP). Subsequent reduction of the resulting nitriles with diisobutyl aluminium hydride (DIBAL-H) hence led to the desired aldehydes with a total yield of 52-53% and an enantiomeric excess (e.e.) of 90%. The original 4-step process developed by Enders and Backes, while being elegant, presented significant limitations for industrial scale production.

Olfactory evaluation of the Helional enantiomers has demonstrated marked differences in odour quality and intensity, rendering both these enantiomers very attractive. Whereas the (S)-Helional® odour has been described as floral, green, marine, ozone- and cumin-like with an odour threshold of 0.64 ng/L, the odour of the other enantiomer, namely (R)-Helional®, has been described as floral (cyclamen/muguet) with aldehyde and citrus-like notes with an odour threshold of 3.43 ng/L. These different olfactory qualities indicate the Helional® enantiomers to be two different and very appreciated fragrances whose blends can give rise to interesting fragrant nuances.

There is hence a felt need to provide both the enantiomers of Helional® and/or enriched blends thereof.

The helional molecule, however, exhibits a chiral centre adjacent to the carbonyl group which can easily undergo racemization if strong bases or acids are used or the operating conditions are insufficiently mild.

The object of the present invention is therefore to provide the two enantiomers while avoiding the easily triggered racemization during the preparation process.

### SUMMARY OF THE INVENTION

The above indicated object was achieved by a process for preparing enriched enantiomers of the compound 3-(benzo[1,3]dioxol-5-yl)-2-methylpropanal (1) of formula 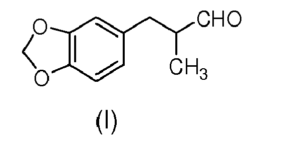 comprising the following steps:
i) reacting the racemic compound 3-(benzo[1,3]dioxol-5-yl)-2-methylpropanol (2) with phthalic anhydride to give the racemic acid (3) 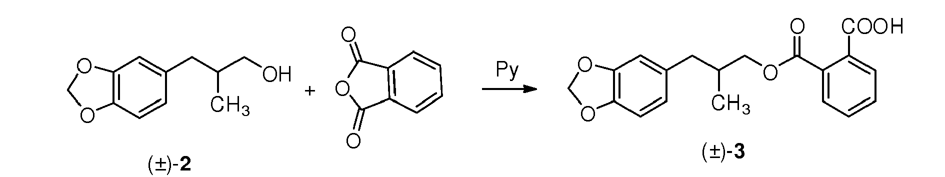
ii) resolving the racemic acid (3) with an optically active 1-aryl-1-ethanamine (4) in which Ar is an aromatic or heteroaromatic group having from 6 to 12 members optionally substituted with one or more substituents chosen from the group consisting of (C₁-C₃) alkyl, (C₁-C₃) alkoxy and halogen 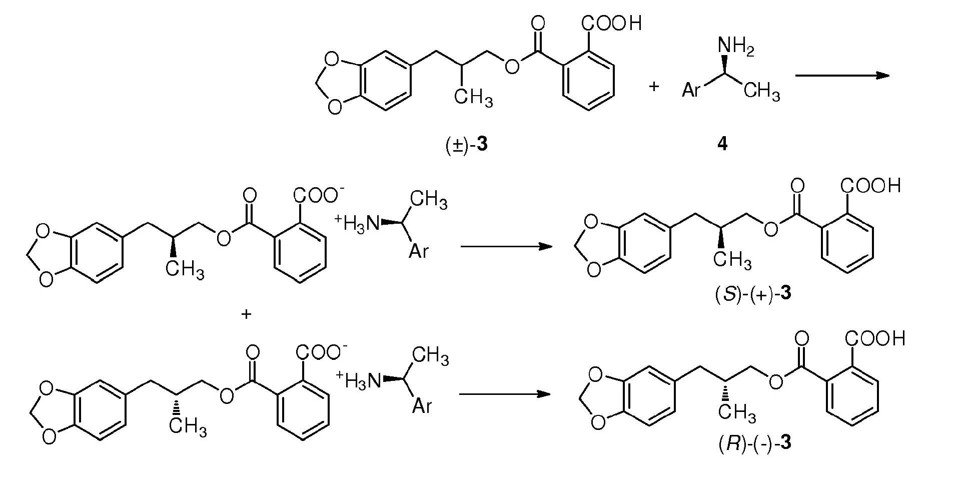
iii) recovering the (S)-(2) and (R)-(2) alcohols from the resolved acids (3) of step ii) by means of alkaline hydrolysis 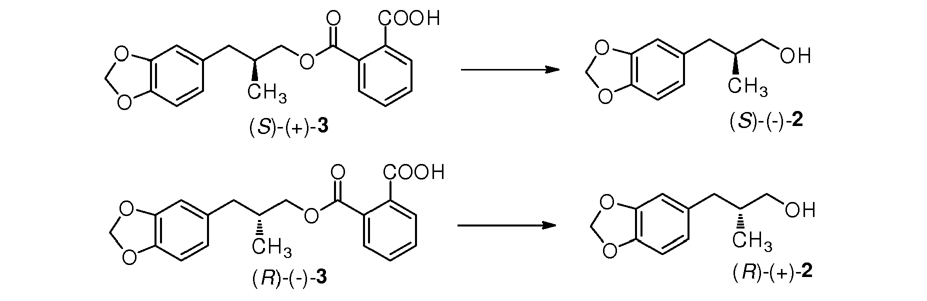
iv) converting the (S) and (R) enantiomers of the alcohol (2) into the respective (S) and (R) enantiomers of compound (1).

Surprisingly the inventors of the present invention have discovered that by resolving the acid (3) they avoided the drawbacks of the prior art. In this respect, the resolved enantiomers of the acid (3) can be effectively converted into the respective enantiomers of the alcohol (2) which can themselves be subsequently converted into the enantiomers of Helional® itself, thus avoiding the easily triggered racemization of the prior methods.

### DESCRIPTION OF THE FIGURES

The invention will now be described in detail with reference to the accompanying figures in which:
Figure 1 shows a summarized scheme of the example of racemic phthalic acid monoester (±)-3 resolution with 1-(4-methoxyphenyl)ethanamine (MOPEA);
Figure 2 shows a summarized scheme of the example of racemic phthalic acid monoester (±)-3 resolution with 1-(4-methylphenyl)ethanamine (MePEA);
Figure 3 shows a summarized scheme of the example of racemic phthalic acid monoester (±)-3 resolution with 1-phenylethanamine (PEA).

### DETAILED DESCRIPTION OF THE INVENTION

The invention therefore relates to a process for preparing enriched enantiomers of the compound 3-(benzo[1,3]dioxol-5-yl)-2-methylpropanal (I) of formula 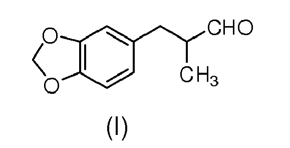 comprising the following steps:
i) reacting the racemic compound 3-(benzo[1,3]dioxol-5-yl)-2-methylpropanol (2) with phthalic anhydride to give the racemic acid (3) 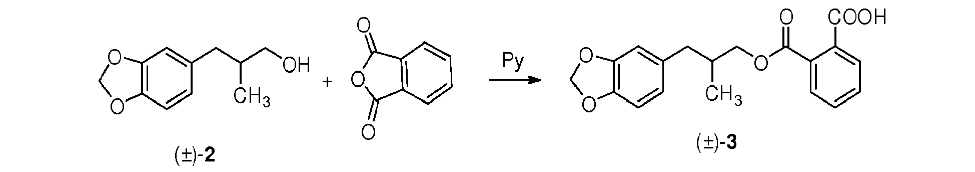
ii) resolving the racemic acid (3) with an optically active 1-aryl-1-ethanamine (4) in which Ar is an aromatic or heteroaromatic group having from 6 to 12 members optionally substituted with one or more substituents chosen from the group consisting of (C₁-C₃) alkyl, (C₁-C₃) alkoxy and halogen. 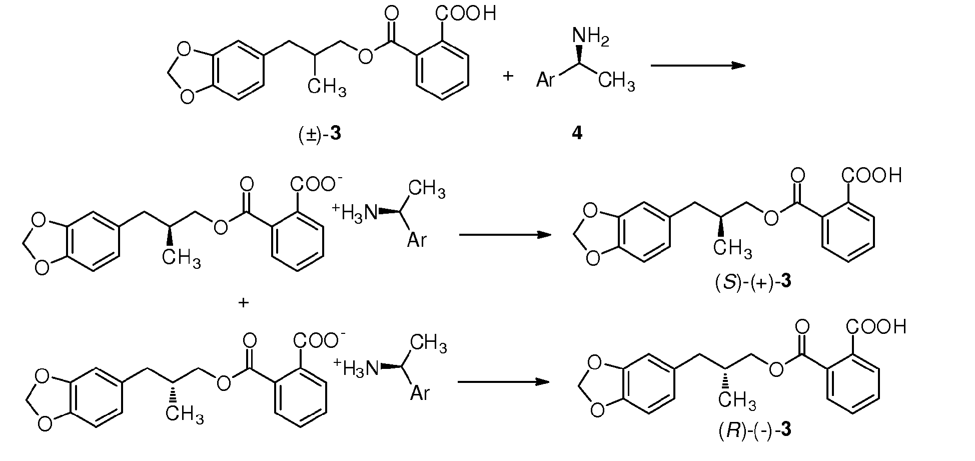
iii) recovering the (S)-(2) and (R)-(2) alcohols from the resolved acids (3) of step ii) by means of alkaline hydrolysis 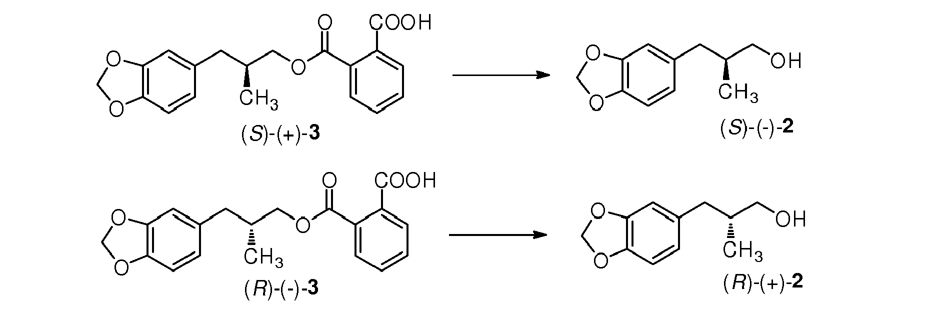
iv) converting the (S) and (R) enantiomers of the alcohol (2) into the respective (S) and (R) enantiomers of compound (1).
Step i) of the process of the invention comprises the reaction between the racemic compound (2) and phthalic anhydride. Said reaction preferably takes place in the presence of pyridine at a temperature within the range from 0 ºC to 90 ºC.
The racemic acid monoester (3) obtained appears advantageously as a pure solid to be used in the resolution step ii) without requiring purification steps.
Step ii) is the step of resolving the acid into the two enantiomers by using an optically active 1-aryl-1-ethanamine (4) in which Ar is an aromatic or heteroaromatic group having from 6 to 12 members optionally substituted with one or more substituents chosen from the group consisting of (C₁-C₃) alkyl, (C₁-C₃) alkoxy and halogen.

Ar is preferably a substituent chosen from the group consisting of phenyl, substituted phenyl, benzodioxolyl, substituted benzodioxolyl. If substituted, the aromatic or heteroaromatic ring is preferably substituted with a substituent chosen from the group consisting of methyl, ethyl or methoxy. More preferably, the 1-aryl-1-ethanamine (4) is chosen from the group consisting of 1-phenylethanamine (PEA), 1-(4-methoxyphenyl)ethanamine (MOPEA), 1-(4-methylphenyl)ethanamine (MePEA) and 1-(benzo[d][1,3]dioxol-5-yl)ethanamine (BDEA).

Another aspect of the invention relates to a diastereoisomeric salt of an enantiomer of the phthalic acid monoester (3) 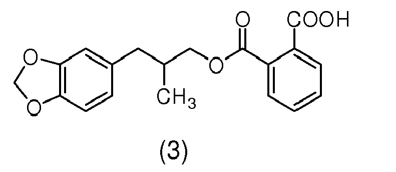 and an optically active 1-aryl-1-ethanamine (4) 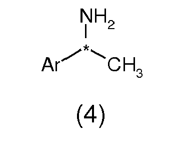 in which Ar is an aromatic or heteroaromatic group having from 6 to 12 members optionally substituted with one or more substituents chosen from the group consisting of (C₁-C₃) alkyl, (C₁-C₃) alkoxy and halogen.

One enantiomer of the acid (3) will be mainly present in the diastereoisomeric salt, and the other in the crystallization waters. The enantiomers of the resolved acid in step iii) are converted into the respective alcohols by alkaline hydrolysis. Said alkaline hydrolysis is preferably conducted with KOH in methanol.

Then in step iv), the (R) and (S) alcohols are converted into the corresponding (R) and (S) aldehydes, namely the enantiomers of 3-(benzo[1,3]dioxol-5-yl)-2-methylpropanal (1). Said reaction can be conducted by using a NaClO solution preferably at a pH between 8.5 and 9.5 in the presence of potassium bromide and a suitable catalyst such as 2,2,6,6-tetramethylpiperidine-1-oxyl (TEMPO; P. L. Anelli, F. Montanari, S. Quici. Org. Synth.; Coll. Vol. 8: 367) or PIPO (A. Dijksman, I. W. C. E. Arends and R. A. Sheldon, Chem. Commun., 2000, 271).
By means of the process of the invention, enriched (R) and (S) enantiomers of 3-(benzo[1,3]dioxol-5-yl)-2-methylpropanal are obtained in good yields, while avoiding the racemization problems of the prior art as will be evident from the following non-limiting examples of the invention.

### Examples

### Example 1

### Preparation of the phthalic acid monoester (±)-3, indicated in brief as (±)-PAM

The reaction of the racemic alcohol 2 (80.00 g, 412 mmol) obtained from the corresponding aldehyde, such as described by Bogert M et al. in JACS (1937), 53, 2747-55 with phthalic anhydride (61.01 g, 412 mmol), was conducted in the presence of pyridine (35.8 g, 453 mmol) without solvent and under stirring, at room temperature (6-8 h) then at 90 °C for 2 hours. The mixture was cooled and the racemic acid product was recovered by extraction with ethyl acetate (400 ml). The mother liquors were washed with 2 M aqueous H₂SO₄ (2 x 100 ml), then with water, dried over MgSO₄ and finally evaporated under reduced pressure (35 °C/18 mbar).

The racemic phthalic acid monoester (±)-3 was then isolated with a 95% yield as a white solid which was sufficiently pure to be used in the resolution step without further purification. A sample was crystallized from ethyl acetate/n-hexane to provide a compound with m.p. of 92-94 °C.

### Example 2

### Resolution of the racemic phthalic acid monoester (±)-3

Resolution of the racemic phthalic acid monoester (±)-3 was carried out by using enantiomers of a 1-aryl-ethanamine chosen from the following group: 1-phenylethanamine (PEA), 1-(4-methoxyphenyl)ethanamine (MOPEA), 1-(4-methylphenyl)ethanamine (MePEA) and 1-(benzo[d][1,3]dioxol-5-yl)ethanamine (BDEA).

### Example 2a

### Resolution of the racemic phthalic acid monoester (±)-3 with MOPEA

The phthalic acid monoester (±)-3 (40 g, 117 mmol) was dissolved in a mixture of diisopropyl ether/methanol (4:1 v/v; 450 ml) by heating to reflux under stirring. To the hot mixture (55 °C), the (S)-(-)-MOPEA (18.60 g, 116.8 mmol) was then added in portions while maintaining the mixture under agitation. On adding the amine, the temperature rose to 65 °C and then the mixture was again heated to reflux to obtain precipitation of a white salt. The mixture was then maintained at reflux under stirring for a further 20-40 minutes. The mixture was then left to cool to ambient temperature while agitating for another 4-8 hours. The salt was then filtered off and dried at 25 °C/24 mbar to obtain 34.36 g of a white solid (PP1). The mother liquors were then evaporated under reduced pressure to give 24.2 g of a gummy oil (ML). After displacement of the base under the usual conditions, HPLC analyses of a sample of ML and PP1 displayed the following composition: ML residue (S)-3/(R):8 =92:8, PP1 precipitate (S)-3/(R)-3 =24:76. The PP1 precipitate was suspended in diisopropyl ether/methanol (7:3 v/v; 250 ml) and heated to reflux for ten minutes then left to cool to room temperature, maintaining under stirring for another 4-6 hours. The solid was filtered off and dried under reduced pressure to obtain 26.4 g of a salt (PP2). Evaporation under reduced pressure (40 ºC/15 mbar) of the filtrate gave 7.4 g of a residue (WL1). HPLC analyses of the PP2 and WL1 samples after the usual displacement of the base displayed, respectively, the following compositions: (S)-3/(R)-3 = 9 : 91 and (S)-3/(R)-3 = 81 : 19. Washing under hot conditions of the PP2 salt with a diisopropyl ether/methanol mixture (7:3 v/v; 250 ml) gave, after cooling and filtering, 24.00 g of a PP3 salt, whose composition after displacement of the base, was (S)-3/(R)-3 = 4 : 96. Evaporation of the filtrate gave 2.44 g of a residue (WL2) whose composition, after displacement of the base, was (S)-3/(R)-3 = 54 : 46.
The PP3 salt and ML residue were treated separately as reported below.

### Recovery of the free acid (R)-(-)-3

The PP3 salt (24.00 g) was treated with 1 M aqueous HCl (70 ml) and diisopropyl ether (100 ml). Following stirring, the organic phase was separated and the aqueous phase extracted three times with 50 ml of diisopropyl ether. The organic extracts were collected, dried (MgSO₄) and evaporated under reduced pressure (40 º C/15 mbar) to give the free acid (R)-(-)-3 as a viscous orange oil [16.6 g, yield = 99.9%, er = 96 : 4, [α]_{D} -17.4 (c 1.000, CHCl₃)].

### Recovery of the free acid (S)-(+)-3

The same process, when applied to the ML residue (24.2 g), gave the free acid (S)-(+)-3 as a viscous orange oil (16.7 g, yield = 99.5 %, er = 92 : 8).

### Recovery of (S)-(-)-1-(4-methoxyphenyl)ethanamine (MOPEA)

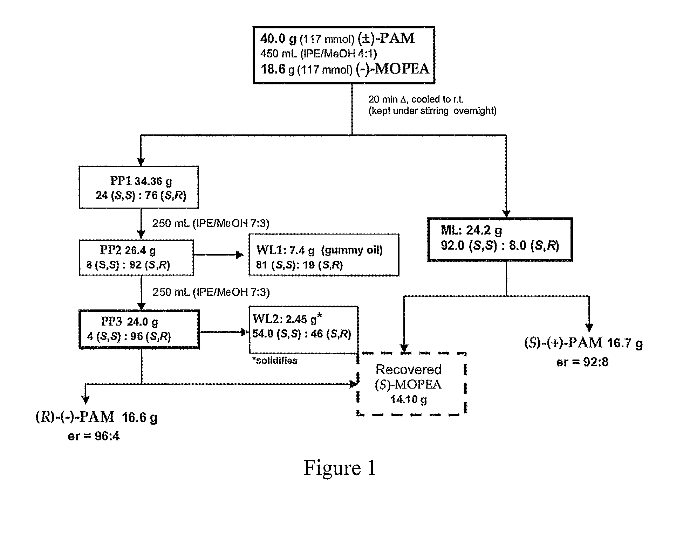

The aqueous phases were collected and treated with 30% aqueous NaOH (%w/v) until the pH was alkaline, then extracted with diisopropyl ether (3 x 80 ml). The organic phases were collected and dried (MgSO₄) and the solvent evaporated under reduced pressure (40 ºC/15 mbar), to give (S)-(-)-1-(4-methoxyphenyl)ethanamine as a colourless oil [14.10 g, yield = 95.5 %, [α]_{D} -33.8 (neat)] which can be used for a further resolution.
The summarized scheme of example 2a is given in Figure 1.

### Example 2b

### Resolution using the enantiomers of MePEA

The racemic phthalic acid monoester (±)-3 (30.0 g, 87.63 mmol) and (S)-(-)-MePEA (11.85 g, 87.6 mmol) were added to a diisopropyl ether/methanol mixture (9:1 v/v; 330 ml) and the mixture heated to reflux. The suspension thus obtained was cooled to room temperature and maintained under stirring for 8-10 hours. The solid thus formed was filtered off and dried at 25 °C under reduced pressure to obtain 23.50 g of a white solid (PP1). The mother liquors were evaporated under reduced pressure (40 ºC/15 mbar) to give 17.61 g of an oil (ML). After the usual displacement of the base, HPLC analyses of ML and PP1 displayed the following composition: ML (S)-3/(R)-3 =89.5 : 10.5; PP1 (S)-3/(R)-3 =22 : 78.
The PP1 solid was suspended in diisopropyl ether/methanol (9:1 v/v; 190 ml) and heated to reflux for ten minutes then left to cool to room temperature and maintained under stirring for 4-8 hours. The solid was filtered off and dried under reduced pressure to obtain 19.45 g of a salt (PP2). Evaporation under reduced pressure (40 ºC/15 mbar) of the precipitation solvent gave 3.98 g of a residue (WL1). HPLC analyses of the PP2 and WL1 samples after the usual displacement of the base displayed, respectively, the following composition (S)-3/(R)-3 = 11 : 89 and (S)-3/(R)-3 = 76.5 : 23.5. Washing PP2 under hot conditions with a diisopropyl ether/methanol mixture (9:1 v/v; 190 ml) gave, after cooling and filtering, 18.14 g of a salt, PP3, whose composition after displacement was (S)-3/(R)-3 = 8.3 : 91.7. Evaporation of the filtrate gave 1.30 g of a residue (WL2) whose composition, after displacement of the base, was (S)-3/(R)-3 = 54 : 46.

### Recovery of the free acid (R)-(-)-3

The PP3 salt [18.14 g, m.p. = 133-134 °C; [α]_{D} -15.9 (c 1.005, CHCl₃)] was treated with 1M aqueous HCl (50 ml) and diisopropyl ether (100 ml). The organic phase was separated and the aqueous phase extracted three times with 50 ml of diisopropyl ether. The organic extracts were collected and dried (MgSO₄). The solvent was evaporated under reduced pressure (40 ºC/15 mbar) to give the free acid (R)-(-)-3 as a viscous orange oil (12.04 g, yield = 99.9%, er = 91.7 : 8:3).

### Recovery of the free acid (S)-(+)-3

The same process was applied to the residue ML [17.62 g; [α]_{D} +3.9 (c 0.75, CHCl₃)] to provide the free acid (S)-(+)-3 as a viscous orange oil (11.85 g, yield = 99.5 %, er = 89.5 : 10.5).

### Recovery of (S)(-)-1-(4-methylphenyl)ethanamine (MePEA)

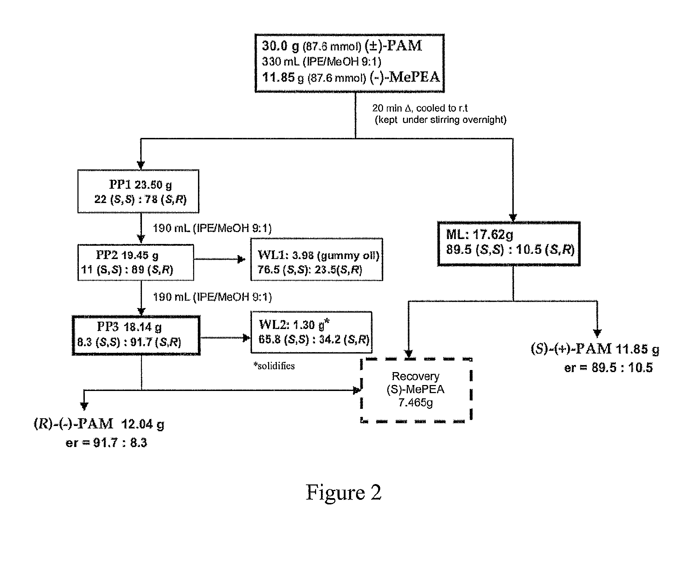

The aqueous phases were collected and treated with aqueous 30% NaOH (% w/v) until the pH was alkaline, then extracted with diisopropyl ether (3 x 80 ml). The organic extracts were collected and dried (MgSO₄) and the solvent evaporated under reduced pressure (40 ºC/15 mbar), to give (S)-(-)-1-(4-methylphenyl)ethanamine as a colourless oil [7.46 g, yield = 81 %, [α]_{D} -29.8 (c 1.339, CHCl₃)] which can be used for a further resolution.
The summarized scheme of example 2b is given in Figure 2.

### Example 2c

### Resolution using the enantiomers of PEA in i-Pr₂O

The racemic phthalic acid monoester (±)-3 (10.0 g, 29.21 mmol) and diisopropyl ether (100 ml) were heated to reflux under stirring. To the hot solution, (S)-(-)-PEA (1.77 g, 14.6 mmol) was added in portions, under stirring and heating was stopped. The mixture was then heated at reflux with formation of an oil. The solution was cooled to room temperature under stirring to obtain a white solid. The solid was then filtered off and dried at 25 ºC under reduced pressure to obtain 6.3 g of PP1. The mother liquors were evaporated at reduced pressure (40 ºC/15 mbar) to give 5.30 g of an oil (ML).
After the usual displacement of the base, HPLC analyses of a sample of ML and PP1 displayed the following respective compositions: ML: (S)-3 (from the n salt)/(R)-3 (from the p salt) = 80 : 20; PP1: (S)-3/(R)-3 = 35 : 65.

### Example 2d

### Resolution using the enantiomers of PEA in MeOH/H₂O

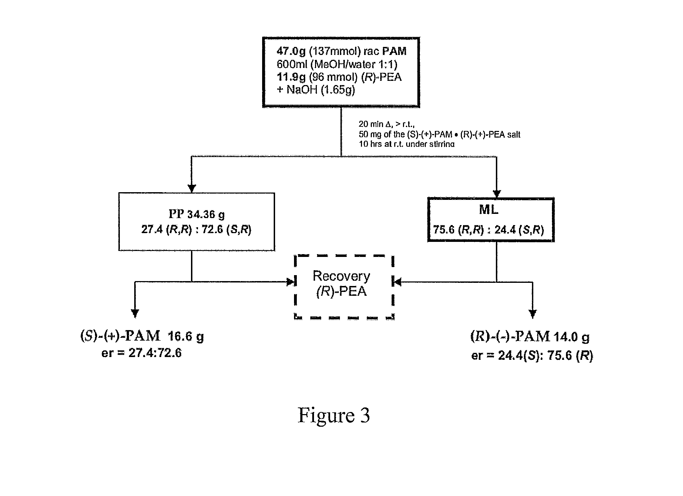

The racemic phthalic acid monoester (±)-3 (47.0 g, 137 mmol) was dissolved in a MeOH/water mixture (3:1 v/v; 400 ml), heating to 65 ºC under stirring. (R)-(+)-PEA (11.9 g, 96 mmol) was added to this mixture under stirring and heating was stopped. At the end of the addition, the mixture was maintained at 35 ºC under stirring after which a solution of NaOH (1.65 g) in water (100 ml) was added. The solution was cooled to room temperature under stirring, then 50 mg of the pure (S)-(+)-PAM • (R)-(+)-PEA salt were added. After a certain amount of time a white solid precipitated. Stirring was continued for a further 6-8 hours and the solid filtered off. The solid was collected and dried at 25 ºC under reduced pressure to obtain 36.4 g of PP, whose composition, after the usual displacement of the base, was (S)-3/(R)-3 = 72.6 : 27.4. The mother liquors (ML) were evaporated under reduced pressure. After evaporation of the methanol, the aqueous suspension was acidified with aqueous 10% HCl (% w/v) and extracted with ethyl acetate. The organic phase was dried (MgSO₄) and evaporated under reduced pressure (40 ºC/15 mbar) to give 14.0 g of a residue whose composition was (S)-3/(R)-3 = 24.3 : 75.7. The PP solid (34.36 g) was dissolved in MeOH/water (2:1 v/v; 185 ml), then the mixture was heated to 65 ºC under stirring. The mixture was cooled to room temperature and maintained under stirring for a further 3-4 hours, then filtered off and the solid obtained dried at 25 ºC under reduced pressure to obtain PP1 (22.3 g). After the usual displacement of the base of the PP1 precipitate and ML2 residue, HPLC analyses of the free acid displayed, respectively, the composition (S)-3/(R)-3 = 85.5 : 14.5 and (S)-3/(R)-3 = 65.5 : 34.5. A second crystallization of PP1 (22.3 g) with MeOH/water (2:1 v/v; 95 ml) gave 17.3 g of a precipitate (PP3) which provided an acid, whose enantiomeric ratio was (S)-3/(R)-3 = 92 : 8.
The summarized scheme of example 2c is given in Figure 3.

### Example 3

### Preparation of (S)-(-)-3-(benzo[d][1,3]dioxol-5-yl)-2-methylpropan-1-ol [(S)-2] by alkaline hydrolysis of the acid monoester (S)-(+)-3

KOH (8.21 g, 146.3 mmol) was added to a solution of acid monoester (S)-(+)-3 (16.7 g, 48.8 mmol) in methanol (90 ml). The mixture was stirred and heated slowly to reflux as KOH dissolved. The mixture was then left under stirring and analyzed by TLC (eluent CH₂Cl₂/MeOH = 9:1 v/v). During the reaction, a white solid precipitated which consisted of the dipotassium salt of phthalic acid. After 1.5 hours the reaction was complete. The solid was filtered off and the methanol removed under reduced pressure (40 ºC/15 mbar); the residue was dissolved in CH₂Cl₂ (150 ml) and washed in an aqueous 10% (% w/v) NaOH solution (50 ml). The aqueous phases were extracted with CH₂Cl₂ (3 x 80 ml) and the organic phases collected , washed with water (3 x 20 ml) then once with a saturated NaCl solution (30 ml), dried (MgSO₄) and evaporated under reduced pressure (40 ºC, 15 mbar) to give a yellow oil (8.385 g). The residue was distilled under reduced pressure (kugelrohr, 185 ºC, 1 mmHg) to give a top fraction (1.460 g of a yellow oil) and a subsequent fraction consisting of 6.732 g of (S)-(-)-3-(benzo[*d*][I,3]dioxol-5-yl)-2-methylpropan-1-ol [(S)-2] as a colourless oil with an e.r. of 92:8, being unchanged relative to the initial (S)-3 acid monoester.

### Preparation of (R)-(+)-3-(benzo[d][1,3]dioxol-5-yl)-2-methylpropan-1-ol [(R)-2] by alkaline hydrolysis of the (R)-3 acid monoester

Using the previous process, the (R)-3 phthalic acid monoester (16.59 g, 48.5 mmol) gave (R)-(+)-3-(benzo[*d*][1,3]dioxol-5-yl)-2-methylpropan-1-ol (9.337 g; after kugelrohr distillation, er = 96 : 4, being unchanged relative to the (R)-3 phthalic acid monoester).

### Example 4

### Oxidation of the alcohol (S)-2 to (S)-Helional (1) by {Poly[[6-[(1,1,3,3-tetramethylbutyl)amino]-1,3,5-triazine-2,4-diyl][(2,2,6,6-tetramethyl-1-oxy-4-piperidinyl)imino]-1,6-hexanediyl[(2,2,6,6-tetramethyl-1-oxy-4-piperidinyl)imino]]} (PIPO)

A solution of KBr (61 mg, 0.51 mmol) in 1 ml of water was added to a solution of PIPO (49 mg) and alcohol (S)-2 (ee 95%; 1.000 g, 5.15 mmol) in CH₂Cl₂ (2 ml). The suspension was vigorously stirred and cooled to 0-4 ºC. A NaClO solution (10.5% w/v; 3.2 ml at pH 9.5 obtained by adding NaHCO₃ to the commercial solution) was added very slowly, ensuring the temperature of the solution did not exceed 5 ºC.
At the end of the addition, GC analysis still showed the presence of the starting alcohol. Hence at various times, a total additional 3.2 ml of the 10.5% NaClO solution at pH 9.5, were added. Subsequent GC analysis showed the disappearance of the starting alcohol. A 10% ascorbic acid solution (w/v; 6.5 ml) was added to the reaction mixture which was left under agitation for 15 minutes followed by addition of 3.2 ml of water. The precipitate was filtered off onto a celite layer and washed with ethyl acetate. The organic phase of the filtrate was separated from the aqueous phase and the latter was extracted three times with ethyl acetate. The pooled organic phases after drying were concentrated under reduced pressure (40 ºC/15 mbar) to obtain (S)-Helional® as a pale yellow oil (0.78 g; yield = 78%).
For the enantiomeric excess determination, a portion of the Helional® obtained was reduced with NaBH₄ in methanol to give the corresponding alcohol. HPLC analysis of the reduction product gave an enantiomeric excess of 95%.

### THE PRODUCTS AND THEIR PHYSICOCHEMICAL PROPERTIES

Phthalic acid monoester (±)-3, i.e. 2-[(3-(benzo[*d*][I,3]dioxol-5-yl)-2-methylpropoxy]carbonyl)benzoic acid: m.p. 93-94 °C. IR (KBr) νₘₐₓ(cm⁻¹): 2957, 2900, 2680, 2563, 1722, 1693, 1601, 1581, 1493, 1445, 1417, 1302, 1258, 1130, 1036, 972, 930, 798, 746. ¹H NMR (300 MHz, CDCl₃), δ: 0.95 (d, J = 6.8 Hz, 3H); 2.04-2.25 (m, 1H); 2.40 (dd, J = 8.15/13.5 Hz, 1H); 2.70 (dd, J = 6.31/13.5 Hz, 1H); 4.15 (dd, J = 6.35/10.8 Hz, 1H); 4.21 (dd, J= 5.90/10.7 Hz, 1H); 5.87 (s, 1H); 6.59 (dd, J= 1.71/7.86 Hz, 1H); 6.64 (d, J = 1.67 Hz, 1H); 6.68 (d, J = 7.84 Hz, 1H); 7.51-7.65 (m, 2H); 7.66-7.73 (m, 1H); 7.82-7.90 (m, 1H); 12.09 (s, H). ¹³C NMR (75 MHz, CDCl₃) δ: 16.52, 34.59, 39.39, 70.11, 100.67, 108.02, 109.38, 121.92, 128.73, 129.74, 129.97, 130.78, 132.11, 133.29, 133.57, 145.70, 147.45, 168.03, 172.67. *m*/*z* 341 (M⁻¹).

Phthalic acid monoester (±)-3, i.e. (S)-(+)-2-[(3-(benzo[*d*][I,3]dioxol-5-yl)-2-methylpropoxy]carbonyl)benzoic acid: thick oil; [α]_{D} +16.4 (c 0.994, CHCl₃). HPLC analysis: (Daicel AD-H, 250x4.6 mm, 254 nm, 25 °C, flow 1.0 mL/min, *n*-hexane/ *i-*Pr₂O = 4:1 + 0.4% acetic acid): *t_{R}* = 10.39 min. Spectroscopic data (IR, ¹H NMR, ¹³C NMR and mass) were identical to those recorded for the racemic compound. Phthalic acid monoester (-)-3, i.e. (R)-(-)-2-[(3-(benzo[*d*][I,3]dioxol-5-yl)-2-methylpropoxy]carbonyl)benzoic acid: thick oil; [α]_{D} -16.38, (c 0.988, CHCl₃). HPLC analysis: (Daicel AD-H, 250x4.6 mm, 254 nm, 25 °C, flow 1.0 mL/min, n-hexane/ *i*-Pr₂O = 4:1 + 0.4% acetic acid): *t_{R}* = 11.64 min. Spectroscopic data (IR, ¹H NMR, ¹³C NMR and mass) were identical to those recorded for the racemic compound.

(R)-(-)-PAM • (S)-(-)-MOPEA [(-)-*p* salt]: m.p. 147-149 °C; [α]_{D} -17.3 (c 1.011, CHCl₃); de 97.8 %. IR (KBr) νₘₐₓ(Cm⁻¹): 3423, 2926, 2875, 2835, 2685, 2645, 2534, 1722, 1628, 1613, 1542, 1518, 1487, 1439, 1396, 1267, 1249, 1190, 1140, 1081, 1039, 932, 803, 761. ¹H NMR (600 MHz, CDCl₃), δ: 0.86 (d, J = 6.70 Hz, 3H); 1.44 (d, J = 6.94 Hz, 3H); 2.06 (m, 1H); 2.30 (dd, J = 8.33/13.65 Hz, 1H); 2.64 (dd, J = 6.03/13.65 Hz, 1H); 3.70 (s, 3H); 3.93 (dd, J = 6.31/10.68 Hz, 1H); 4.00 (dd, J = 6.21/10.68 Hz, 1H); 4.17 (q, J = 6.94 Hz, 1H); 5.85 (s, 2H); 6.54 (dd, J = 1.78/7.97 Hz, 1H); 6.61 (d, J= 1.78, 1H); 6.66 (m, 3H); 7.20 (m, 2H); 7.28 (m, 2H); 7.32 (m, 1H); 7.56 (m, 1H); 8.02 (bs, 3H) ppm. ¹³C NMR (150 MHz, CDCl₃) δ: 16.47, 21.25, 34.72, 39.45, 50.51, 55.06, 69.11, 100.69, 108.01, 109.47, 113.91, 121.96, 127.84, 128.17, 128.40, 130.58, 130.83, 132.20, 133.81, 139.58, 145.68, 147.44, 159.11, 168.67, 173.90 ppm. The mass spectra (m/z) were obtained in the electrospray (ESI) mode: ESI-MS: 341 [C₁₉H₁₇O₆]⁻, ESI-MS: 152 [C₉H₁₄NO]⁺

(S)-(+)-PAM • (S)-(-)-MOPEA [(+)-*n* salt]: gummy oil; [α]_{D} +2.42 (c 1.03, CHCl₃); de 86%. IR (CCl₄) νₘₐₓ(cm⁻¹): 2934, 1725, 1607, 1585, 1560, 1518, 1490, 1442, 1379, 1251, 1076, 1042, 1006, 979. ¹H NMR (600 MHz, CDCl₃), δ: 0.87 (d, J = 6.73 Hz, 3H); 1.42 (d, J = 6.88 Hz, 3H); 2.05 (m, 1H); 2.30 (dd, J = 8.34/13.64 Hz, 1H); 2.64 (dd, J = 6.14/13.64 Hz, 1H); 3.69 (m, 2H); 4.17 (q, J = 6.88 Hz, 1H); 5.85 (s, 2H); 6.54 (dd, J = 1.76/7.90 Hz, 1H); 6.61 (d, J = 1.76 Hz, 1H); 6.63 (d, J = 7.90 Hz, 1H); 6.66 (d, J = 7.90 Hz, 1H); 7.18 (m, 2H); 7.22 (dd, J = 1.20/7.56 Hz, 1H); 7.26 (ddd, J = 1.20/7.35/7.64 Hz, 1H); 7.31 (ddd, J = 1.48/7.35/7.51 Hz, 1H); 7.56 (dd, J = 1.48/7.56 Hz, 1 H); 8,21 (bs, 3H) ppm. ¹³C NMR (150 MHz, CDCl₃) δ: 16.46, 21.02, 34.72, 39.42, 50.43, 55.01, 69.03, 100.66, 107.98, 109.41, 113.86, 121.91, 127.79, 127.95, 128.27, 130.54, 130.63, 131.79, 133.79, 139.99, 145.65, 147.42, 159.08, 168.57, 174.12 ppm. The mass spectra (m/z) were obtained in the electrospray (ESI) mode: ESI-MS: 341 [C₁₉H₁₇O₆]⁻; ESI-MS: 152 [C₉H₁₄NO]⁺

(S)-(+)-PAM • (R)-(+)-MOPEA [(+)-*p* salt]: m.p. 147-149 °C; [α]_{D} +17.2 (c 1.00, CHCl₃); de 97.5 %. The spectroscopic data (IR, ¹ NMR, ¹³C NMR and mass) were identical to those recorded for the levorotatory enantiomer.

(S)-(-)-PAM • (S)-(+)-MOPEA [(-)-*n* salt]: gummy oil; [α]_{D} -2.38 (c 1.00, CHCl₃); de 97.5 %. The spectroscopic data (IR, ¹H NMR, ¹³C NMR and mass) were identical to those recorded for the dextrorotatory enantiomer.

(S)-(+)-PAM • (R)-(+)-PEA [(+)-*p* salt]: m.p. 146-148 °C; [α]_{D} +15.6 (c 1.000, CHCl₃). IR(KBr) νₘₐₓ(cm⁻¹): 3448, 2979, 2885, 2537, 1719, 1629, 1542, 1487, 1399, 1388, 1263, 1245, 1138, 1076, 1038, 983, 939, 924, 863, 793, 759, 703. ¹H NMR (300 MHz, CHCl₃), δ: 0.86 (d, J= 6.78 Hz, 3H); 1.45 (d, J=6.83 Hz, 3H); 1.93-2.15 (m, 1H); 2.31 (dd, J = 8.27/13.58 Hz, 1H); 2.64 (dd, J = 5.96/13.58 Hz, 1H); 3.97 (dq, J = 6.21/10.82 Hz, 2H); 4.15-4.27 (q, J = 6.83 Hz, 1H); 5.85 (s, 2H); 6.54 (dd, J = 1.69/7.92 Hz, 1H); 6.60 (d, J = 1.64 Hz, 1H); 6.66 (d, J = 7.88 Hz, 1H); 7.08-7.36 (m, 8 H); 7.53-7.59 (m, 1H); 7.65 (bs, 3H) ppm. ¹³C NMR (75 MHz, CHCl₃) 5:16.49, 21.37, 34.70, 39.46, 51.05, 69.05, 100.69, 108.02, 109.46, 121.94, 126.57, 127.83, 127.93, 128.11; 128.27; 128.61, 130.71, 133.80, 139.76, 140.11, 145.68, 147.44, 168.55, 174.10 ppm. The mass spectra (m/z) were obtained in the electrospray (ESI) mode: ESI-MS: 341 [C₁₉H₁₇O₆]⁻; ESI-MS: 136[C₉H₁₄N]⁺.

(R)-(-)-PAM • (R)-(+)-PEA [(-)-*n* salt]: m.p. 115-116 °C; [α]_{D} -8.5 (c 1.000, CHCl₃).IR (KBr) (cm-1): 3448, 2936, 2680, 2534, 1717, 1626, 1528, 1503, 1490, 1388, 1387, 1259, 1134, 1074, 1036, 928, 800, 763, 695. ¹H NMR (300 MHz, CHCl₃), δ: 0.86 (d, J = 6.73 Hz, 3H); 1.44 (d, J = 6.75 Hz, 3H); 1.93-2.13 (m, 1H); 2.29 (dd, J = 8.29/13.5 Hz, 1H); 2.63 (dd, J = 6.07/13.5 Hz, 1H); 3.85-4.00 (m, 2H); 4.18 (q, J = 6.25 Hz, 1H); 5.83 (s, 2H); 6.54 (dd, J = 7.85/1.61 Hz, 1H); 6.66 (d, J = 7.84 Hz, 1H); 7.05-7.34 (m, 8H); 7.49-7.58 (m, 1H); 8.72 (bs, 3H) ppm. ¹³C NMR (75 MHz, CHCl3) δ: 16.46, 21.24, 34.70, 39.41, 50.97, 68.91, 100.64, 107.96, 109.39, 121.89, 126.57, 127.75, 127.78, 127.93, 128.19, 128.54 130.57, 133.78, 139.82, 139.93, 145.64, 147.41, 168.43, 174.15 ppm. The mass spectra (m/z) were obtained in the electrospray (ESI) mode: ESI-MS: 341 [C₁₉H₁₇O₆]⁻; ESI-MS: 122 [C₉H₁₄N]⁺.

(R)-(-)-PAM • (S)-(-)-PEA [(-)-*p* salt]: m.p. 146-148 °C; [α]_{D} -15.5 (c 1.000, CHCl₃). The spectroscopic data (IR, ¹H NMR, ¹³C NMR and mass) were identical to those recorded for the dextrorotatory enantiomer.

(S)-(+)-PAM • (S)-(-)-PEA [(+)-*n* salt]: m.p. 115-116 °C; [α]_{D} +8.5 (c 1.005, CHCl₃). The spectroscopic data (IR, ¹H NMR, ¹³C NMR and mass) were identical to those recorded for the levorotatory enantiomer.

(R)-(-)-PAM • (S)-(-)-MePEA [(-)-*p* salt: m.p. 136-138 °C; [α]_{D} -16.6 (c 0.996, CHCl₃). IR (KBr), νₘₐₓ (cm-1): 2968, 2916, 1720, 1542, 1488, 1439, 1397, 1274, 1247, 1190, 1142, 1082, 1040, 996, 942. aH NMR (300 MHz, CHCl3), S: 0.86 (d, 3H, J = 6.74 Hz); 1.43 (d, 3H, J = 6.94 Hz); 1.93-2.11 (m, 1 H); 2.23 (s, 3H); 2.29 (dd, 1H, J = 8.16/13.69 Hz); 2.64 (dd, 1H, J = 5.83/13.52 Hz); 3.91 (dd, 1H, J = 6.42/10.74 Hz); 4.00 (dd, 1H, J = 6.31/10.74 Hz); 4.17 (q, 1H, J = 7.00 Hz); 5.85 (s, 2H); 6.54 (dd, 1H, J = 1.69/7.84 Hz); 6.60 (d, 1H, J = 1.60 Hz); 6.66 (d, 1H, J = 7.84 Hz); 6.90 (s, 1H); 6.93 (s, 1H); 7.10-7.35 (m, 5H); 7.55 (d, 1H, J = 7.56 Hz); 8.22 (bs, 3H) ppm. ¹³C NMR (75 MHz, CHCl3) δ: 16.43, 21.04, 21.20, 34.69, 39.43, 50.72, 69.00, 100.66, 107.98, 109.43, 121.91, 126.53, 127.75, 127.91, 128.27, 129.21, 130.47, 130.65, 133.78, 136.91, 137.30, 139.92, 145.65, 147.41, 168.57, 174.08 ppm. The mass spectra (m/z) were obtained in the electrospray (ESI) mode: ESI-MS: 341 [C₁₉H₁₇O₆]⁻; ESI-MS: 136 [C₉H₁₄N]⁺.

(S)-(+)-PAM • (S)-(-)-MePEA [(+)-*n* salt]: m.p. 84-86 °C; [α]_{D} +5.8 (e 0.998, CHCl₃). IR (KBr), νₘₐₓ (cm⁻¹): 2968, 2917, 1721, 1624, 1542, 1488, 1440, 1396, 1385, 1273, 1247, 1190, 1141, 1081, 1039, 996, 942. ¹H NMR (300 MHz, CHCl₃), δ:0.86 (d, J = 6.72 Hz, 3H); 1.43 (d, J = 6.87 Hz, 3H); 1.96-2.11 (m, 1H); 2.24 (s, 3H); 2.28 (dd, J = 8.40/13.5 Hz, 1H); 2.64 (dd, J = 5.92/13.5 Hz, 1H); 3.95 (d, J = 6.14 Hz, 2H); 4.16 (q, J = 6.64 Hz, 1H); 5.84 (s, 2H); 6.54 (dd, J = 1.62/7.90 Hz, 1H); 6.60 (d, J = 1.47, 1H); 6.66 (d, J = 7.83 Hz, 1H); 6.91 (d, J = 7.97 Hz, 2H); 7.09-7.35 (m, 5H); 7.49-7.58 (d, J = 7.75 Hz, 1H); 8.56 (bs, 3H) ppm. 13C NMR (75 MHz, CHCl3) δ: 16.43, 21.04, 21.20, 34.72, 39.41, 50.70, 68.95, 100.65, 107.96, 109.40, 121.90, 126.51, 127.69, 127.88, 128.27, 129.20, 130.41, 130.70, 133.79, 136.87, 137.26, 139.90, 145.63, 147.40, 168.55, 174.06 ppm. The mass spectra (m/z) were obtained in the electrospray (ESI) mode: ESI-MS: 341 [C₁₉H₁₇O₆]⁻; ESI-MS: 136 [C₉H₁₄N]⁺.

(S)-(+)-PAM • (R)-(+)-MePEA [(+)-*p* salt]: m.p. 136-138 °C; [α]_{D} +16.5 (c 0.998, CHCl₃). The spectroscopic data (IR, ¹H NMR, ¹³C NMR and mass) were identical to those recorded for the levorotatory enantiomer.

(R)-(-)-PAM • (R)-(+)-MePEA [(-)-*n* salt]: m.p. 84-86 °C; [α]_{D} -5.8 (c 1.000, CHCl₃). The spectroscopic data (IR, ¹H NMR, ¹³C NMR and mass) were identical to those recorded for the dextrorotatory enantiomer.

(R)-(-)-PAM • (S)-(-)-BDEA [(-)-*p* salt]: m.p. 145-146 °C; [α]_{D} -18.6 (c 1.003, CHCl₃). IR (KBr), νₘₐₓ (cm-1): 4422, 2943, 2926, 2686, 2536, 2193, 1718, 1629, 1541, 1490, 1443, 1398, 1388, 1265, 1251, 1189, 1141, 1083, 1039, 945, 878, 814, 804, 758, 708, 694. 1 H NMR (600 MHz, CHCl₃), δ: 0.88 (d, J = 6.75 Hz, 3H); 1.42 (d, J = 6.72 Hz, 3H); 1.93-2.11 (m, 1H); 2.31 (dd, J = 8.27/13.73 Hz, 1H); 2.65 (dd, J = 5.98/13.84 Hz, 1H); 3.97 (dd, J = 6.42/10.82 Hz, 1H); 4.04 (dd, J = 6.42/10.81 Hz, 1H); 4.14 (q, J = 6.75 Hz, 1H); 5.85 (bs, 2H); 5.86 (bs, 2H); 6.54-6.56 (m, 2H); 6.61 (d, J= 1.56 Hz, 1H); 6.66-6.68 (m, 1H); 6.74 (dd, J= 1.65/8.11 Hz, 1H); 6.83 (d, J = 1.52 Hz, 1H); 7.31-7.34 (m, 2H); 7.36-7.38 (m, 1H); 7.55-7.57 (m, 1H); 8.02 (bs, 3H) ppm.¹³C NMR (150 MHz, CHCl3) δ: 16.49, 21.57, 34.73, 39.45, 50.94, 69.14, 100.71, 100.94, 107.23, 108.02, 108.26, 109.47, 120.20, 121.96, 127.89, 128.27, 128.37, 130.58, 130.84, 133.82, 139.29, 145.69, 147.10, 147.44, 147.64, 168.68, 173.86 ppm. The mass spectra (m/z) were obtained in the electrospray (ESI) mode:ESI-MS: 341 [C₁₉H₁₇O₆]⁻; ESI-MS: 166 [C₉H₁₄N]⁺.

(S)-(+)-PAM • (S)-(-)-BDEA [(+)-*n* salt]: yellowish oil which solidifies on evaporation of the solvent: m.p. 37-39 °C; [α]_{D} +1.2 (c 1.151, CHCl₃). IR (KBr), νₘₐₓ (cm-1): 3422, 3051, 2964, 2896, 2770, 1720, 1609, 1587, 1560, 1504, 1490, 1443, 1385, 1250, 1122, 1076, 1038, 925, 812. ¹H NMR (600 MHz, acetone-D₆), δ: 0.98 (d, J = 6.75 Hz, 3H); 1.41 (d, J = 5.87 Hz, 3H); 2.16-2.24 (m, 1H); 2.46 (dd, J = 8.18/13.54 Hz, 1H); 2.79 (dd, J = 6.25/13.49 Hz, 1H); 4.14 (dd, J = 6.36/10.88 Hz; 1H); 4.18 (dd, J = 5.87/10.88 Hz, 1H); 4.71 (q, J = 6.46, 1H); 5.966 (d, J = 1.17 Hz, 1H); 5.970 (d, J = 1.17 Hz, 1H); 5.976 (d, J = 1.17 Hz, 1H); 5.982 (d, J = 1.17 Hz, 1H); 6.71 (dd, J = 1.79/7.93 Hz, 1H); 6.76-6.80 (m, 3H); 6.94 (dd, J = 1.44/7.91 Hz, 1H); 7.05 (d, J = 1.74 Hz, 1H); 7.59-7.61 (m, 3 H); 7.65-7.68 (m, 1H); 7.85-7.88 (m, 1H) ppm. ¹³C NMR (150 MHz, CHCl3) δ: 16.89, 24.73, 35.58, 39.99, 59.47, 70.00, 101.65, 101,73, 108.07, 108.57, 108,67, 110.24, 120.54, 122.91, 128.89, 129.90, 131.11, 131.18, 134.31, 134.99, 135.40, 140.44, 146.71, 147.11, 148.49, 148.53, 169.17, 169.80 ppm. The mass spectra (m/z) were obtained in the electrospray (ESI) mode: ESI-MS: 341 [C₁₉H₁₇O₆]⁻; ESI-MS: 166 [C₉H₁₄N]⁺.

(S)-(-)-3-(benzo[*d*][1,3]dioxol-5-yl)-2-methylpropan-1-ol: as an oil: B. P. 116-117°C (0.27 mmHg); [α]_{D} - 11.5 and 1.033, CHCl₃ HPLC analysis: er = 93:7; ee = 86%

(Daicel OD-H, 250x4.6 mm, 254 nm, 25 °C, 1.0 ml/min, n-hexane/*i*-PrOH = 95:5: t_{R} = 13.70 min). IR (neat), νₘₐₓ(cm⁻¹): 3367, 2956, 2779, 1608, 1491, 1442, 1361, 1247, 1191, 1125, 1101, 1039, 985, 935, 865, 808, 771, 641, 605. ¹H NMR (300 MHz, CDCl₃), δ: 0.90 (d, J= 6.6 Hz, 3H); 1.51 (s, 1H); 1.88 (m, 1H); 2.34 (dd, J = 8.0/13.7 Hz, 1H); 2.67 (dd, J = 6.3/13.5 Hz, 1H); 3.48 (m, 2H); 5.91 (s, 2H); 6.60-6.73 (m, 3H) ppm. ¹³C NMR (75 MHz, CHCl₃) δ: 16.38; 37.84; 39.33; 67.42; 100.59; 107.87; 109.29; 121.71; 134.21; 145.43; 147.28 ppm.

(R)-(+)-3-(benzo[*d*][1,3]dioxol-5-yl)-2-methylpropan-1-ol: as an oil: B.P. 116-117°C (0.27 mmHg); [α]_{D} + 11.8 (c 1.108, CHCl₃). HPLC analysis: er = 98:2; ee = 96%

(Daicel OD-H, 250x4.6 mm, 254 nm, 25 °C, 1.0 ml/min, n- hexane/*i*-PrOH = 95:5: t_{R} = 12.53 min). The spectroscopic data (IR, ¹H NMR, ¹³C NMR and mass) were identical to those recorded for the (S)-(-)-2 enantiomer.

(S)-(-)-3-(benzo[*d*][1,3]dioxol-5-yl)-2-methylporopanal [(S)-11: as an oil: TLC (R_{f}) 0.53 (EP/EA = 8/2); [α]_{D} -4.1 (c 1.3, CHCl₃). ¹H-NMR (400 MHz, CDCl₃) δ (ppm) 9.70 (d, 1H, *J*=1.5 Hz); 6.73 (d, 1H, *J*=7.9 Hz); 6.65 (d, 1H, *J*=1.7 Hz); 6.61 (dd, 1H, *J*=7.9 Hz, *J*=1.7 Hz); 5.93 (s, 2H); 3.00 (dd, 1H, *J*=13.5 Hz, *J*=5.8 Hz); 2.67-2.57 (m, 1H); 2.54 (dd, 1H, *J*=13.5 Hz, *J*=8.0 Hz); 1.08 (d, 3H, *J*=6.9 Hz). HPLC analysis of the alcohol obtained by reduction with NaBH₄: ee = 95% (Daicel OD-H, 250x4.6 mm, 254 nm, 25 °C, 1.0 ml/min, n- hexane/*i*-PrOH = 95:5)

## Claims

1. Process for preparing enriched enantiomers of the compound 3-(benzo[1,3]dioxol-5-yl)-2-methylpropanal of formula 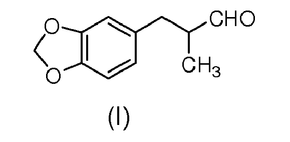 comprising the following steps:
i) reacting the racemic compound 3-(benzo[1,3]dioxol-5-yl)-2-methylpropanol (2) with phthalic anhydride to give the racemic acid (3) 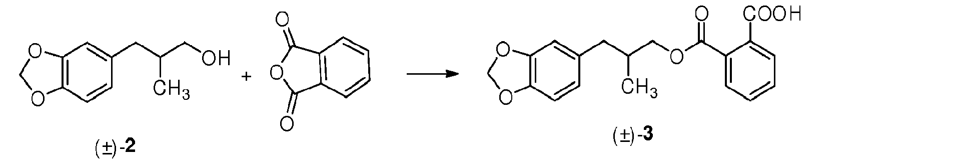
ii) resolving the racemic acid (3) with an optically active 1-aryl-1-ethanamine (4) in which Ar is an aromatic or heteroaromatic group having from 6 to 12 members optionally substituted with one or more substituents chosen from the group consisting of (C₁-C₃) alkyl, (C₁-C₃) alkoxy and halogen 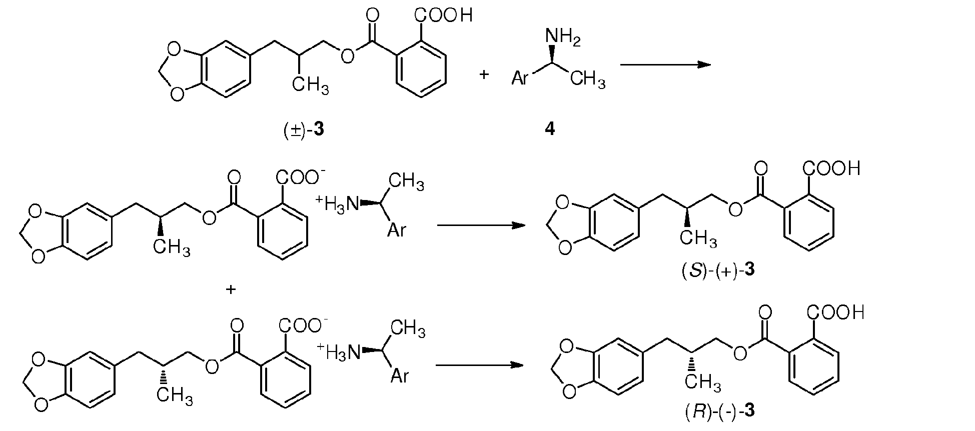
iii) recovering the (S)-(2) and (R)-(2) alcohols from the resolved acids (3) of step ii) by means of alkaline hydrolysis 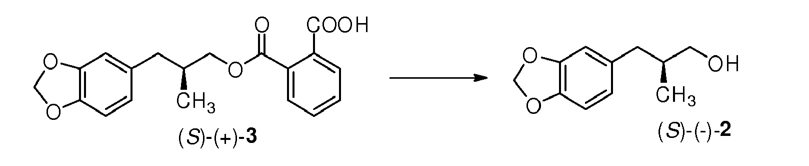 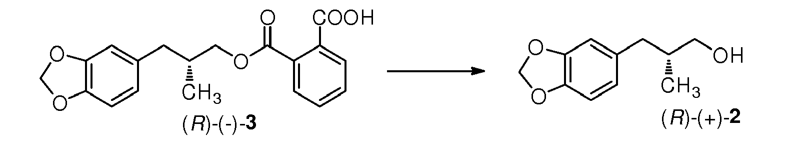
iv) converting the (S)-(2) and (R)-(2) alcohols into the respective (S) and (R) enantiomers of compound (1).

2. Process according to claim 1 wherein in step i) the reaction between the racemic compound (2) and phthalic anhydride takes place in the presence of pyridine at a temperature within the range from 0 ºC to 90 ºC.

3. Process according to claim 1 or 2 wherein in step ii) Ar is a substituent chosen from the group consisting of phenyl, substituted phenyl, benzodioxolyl, substituted benzodioxolyl.

4. Process according to any one of claims 1 to 3 wherein the aromatic or heteroaromatic ring of an optically active 1-aryl-1-ethanamine (4), if substituted, is substituted with a substituent chosen from the group consisting of methyl, ethyl, methoxy.

5. Process according to any one of claims 1 to 4 wherein the 1-aryl-1-ethanamine (4) is chosen from the group consisting of 1-phenylethanamine (PEA), 1-(4-methoxyphenyl)ethanamine (MOPEA), 1-(4-methylphenyl)ethanamine (MePEA) and 1-(benzo[*d*][1,3]dioxol-5-yl)ethanamine (BDEA).

6. Process according to any one of claims 1 to 5 wherein the enantiomers of the acid resolved in step iii) are converted into the respective alcohols by alkaline hydrolysis with KOH in methanol.

7. Process according to any one of claims 1 to 6 wherein step iv) of converting the (R)-2 and (S)-2 alcohols into (R)-1 and (S)-1 aldehydes, is conducted by using an aqueous solution of NaClO, at a pH between 8.5 and 9.5, in the presence of potassium bromide and a suitable piperidinyl-1-oxyl.

8. A diastereoisomeric salt of an enantiomer of the phthalic acid monoester (3) 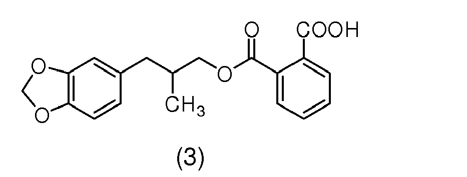 and an optically active 1-aryl-1-ethanamine (4) 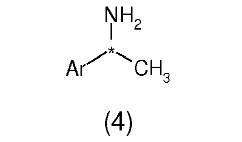 in which Ar is an aromatic or heteroaromatic group having from 6 to 12 members optionally substituted with one or more substituents chosen from the group consisting of (C₁-C₃) alkyl, (C₁-C₃) alkoxy and halogen.

9. A diastereoisomeric salt according to claim 8 wherein Ar of the optically active 1-aryl-1-ethanamine (4) is a substituent chosen from the group consisting of phenyl, substituted phenyl, benzodioxolyl, substituted benzodioxolyl.

10. A diastereoisomeric salt according to claim 8 or 9 wherein the aromatic or heteroaromatic ring of an optically active 1-aryl-1-ethanamine (4), if substituted, is substituted with a substituent chosen from the group consisting of methyl, ethyl, methoxy.

11. A diastereoisomeric salt according to any one of claims 8 to 10 wherein the 1-aryl-1-ethanamine (4) is chosen from the group consisting of 1-phenylethanamine (PEA), 1-(4-methoxyphenyl)ethanamine (MOPEA), 1-(4-methylphenyl)ethanamine (MePEA) or 1-(benzo[*d*][1,3]dioxol-5-yl)ethanamine (BDEA).
